# EUROPÄISCHE PATENTSCHRIFT

(11) **EP 3 502 084 B1**
(45) Veröffentlichungstag und Bekanntmachung des Hinweises auf die Patenterteilung: **12.02.2020**
(21) Anmeldenummer: 17209336.1
(22) Anmeldetag: 21.12.2017
(51) Int. Cl.: C07C 51/14, C07C 53/126, B01J 31/18, B01J 31/22, B01J 31/24, B01J 31/30

(54) **VERFAHREN ZUR DIREKTEN UMSETZUNG VON DIISOBUTEN ZU EINER CARBONSÄURE**
METHOD FOR DIRECT CONVERSION OF DI-ISOBUTENE TO A CARBOXYLIC ACID
PROCÉDÉ DE CONVERSION DIRECTE DU DIISOBUTÈNE EN UN ACIDE CARBONIQUE

(43) Veröffentlichungstag der Anmeldung: 26.06.2019
(73) Patentinhaber: Evonik Operations GmbH, 45128 Essen (DE)
(72) Erfinder: SANG, Rui, 252000 Liaocheng City, Shandong Province (CN); KUCMIERCZYK, Peter, 44652 Herne (DE); DONG, Kaiwu, 236800 Bo Zhou (CN); JACKSTELL, Ralf, 10106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- WO-A1-2014/005854
- DE-C- 887 644
- GB-A- 1 595 037
- US-A- 3 641 074
- US-A- 3 968 133

## Beschreibung

Die Erfindung betrifft ein Verfahren zur direkten Umsetzung von Diisobuten zu einer Carbonsäure.

Carbonsäuren werden bei der Herstellung von Polymeren, Pharmazeutika, Lösungsmitteln und Lebensmittelzusatzstoffen verwendet. Zu den Routen, welche zu Carbonsäuren führen, gehören im Allgemeinen die Oxidation von Kohlenwasserstoffen, Alkoholen oder Aldehyden, die oxidative Spaltung von Olefinen durch Ozonolyse, die Hydrolyse von Triglyceriden, Nitrilen, Estern oder Amiden, die Carboxylierung von Grignard- oder Organolithium-Reagenzien und die Halogenierung und anschließende Hydrolyse von Methylketonen in der Haloform-Reaktion. WO 2014/ 005854 offenbart ein zweistufiges Verfahren von Diisobutylen zu 3,5,5-Trimethylhexansäure.

Der Erfindung lag die Aufgabe zugrunde, ein Verfahren bereitzustellen, mit welchem Diisobuten (DIBN) direkt zu einer Carbonsäure umgesetzt werden kann.

Unter "direktem Umsatz" ist im Rahmen dieser Anmeldung zu verstehen, dass die Reaktion in einem Schritt, also ohne Abtrennung oder Aufarbeitung oder dergleichen eines Zwischenproduktes erfolgt.

Hierbei ist nicht ausgeschlossen, dass sich im Verlauf der Reaktion intermediär Zwischenstufen ausbilden, welche direkt weiter umgesetzt werden.

Gelöst wird die Aufgabe durch ein Verfahren nach Anspruch 1.

Verfahren umfassend die Verfahrensschritte:
a) Zugabe von Diisobuten;
b) Zugabe eines Komplexes, welcher eine Verbindung gemäß der allgemeinen Formel (I) umfasst, sowie Pd,
   oder einer Verbindung gemäß der allgemeinen Formel (**I**) und eine Substanz, welche Pd umfasst 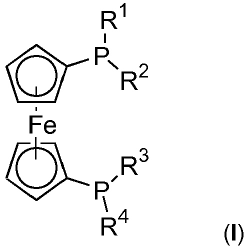 wobei
   R¹, R², R³, R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl;
   mindestens einer der Reste R¹, R², R³, R⁴ für einen -(C₆-C₂₀)-Heteroarylrest mit mindestens sechs Ringatomen steht;
      und
   R¹, R², R³, R⁴, falls diese für -(C₁-C₁₂)-Alkyl, -(C₃-C₁₂)-Cycloalkyl, -(C₃-C1₂)-Heterocycloalkyl, -(C₆-C₂₀)-Aryl, -(C₃-C₂₀)-Heteroaryl oder -(C₆-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl,-COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C3-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können;
c) Zuführen von CO;
d) Erhitzen des Reaktionsgemisches, so dass das Diisobuten zu einer Carbonsäure umgesetzt wird,
   wobei das Diisobuten direkt zur Carbonsäure umgesetzt wird.

In einer Variante des Verfahrens stehen mindestens zwei der Reste R¹, R², R³, R⁴ für einen -(C₆-C₂₀)-Heteroarylrest mit mindestens sechs Ringatomen.

In einer Variante des Verfahrens stehen die Reste R¹ und R³ jeweils für einen -(C₆-C₂₀)-Heteroarylrest mit mindestens sechs Ringatomen.

In einer Variante des Verfahrens stehen die Reste R¹ und R³ jeweils für einen -(C₆-C₂₀)-Heteroarylrest mit mindestens sechs Ringatomen;
R² steht für -(C₆-C₂₀)-Heteroaryl mit mindestens sechs Ringatomen oder ist ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl;
und R⁴ ist ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl.

In einer Variante des Verfahrens stehen die Reste R¹ und R³ jeweils für einen -(C₆-C₂₀)-Heteroarylrest mit mindestens sechs Ringatomen;
und R² und R⁴ sind ausgewählt aus -(C₁-C₁₂)-Alkyl, -(C₃-C₁₂)-Cycloalkyl, -(C₃-C₁₂)-Heterocycloalkyl, -(C₆-C₂₀)-Aryl.

In einer Variante des Verfahrens stehen die Reste R¹ und R³ jeweils für einen -(C₆-C₂₀)-Heteroarylrest mit mindestens sechs Ringatomen;
und R² und R⁴ stehen für -(C₁-C₁₂)-Alkyl.

In einer Variante des Verfahrens sind R¹, R², R³, R⁴, falls diese für einen Heteroarylrest stehen, jeweils unabhängig voneinander ausgewählt aus Pyridyl, Pyridazinyl, Pyrimidyl, Pyrazinyl, Benzofuranyl, Indolyl, Isoindolyl, Benzimidazolyl, Chinolyl, Isochinolyl.

In einer Variante des Verfahrens weist die Verbindung (**I**) die Struktur (1) auf:

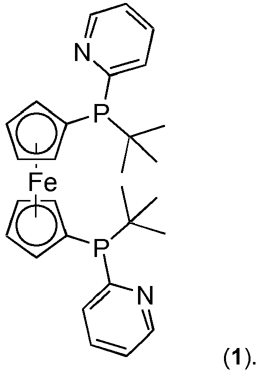

In einer Variante des Verfahrens ist die Substanz im Verfahrensschritt b) ausgewählt aus: PdCl₂, PdBr₂, Pd(acac)₂, Pd(dba)₂ (dba = Dibenzylidenaceton), PdCl₂(CH₃CN)₂.

In einer Variante des Verfahrens ist die Substanz im Verfahrensschritt b) Pd(acac)₂.

In einer Variante des Verfahrens umfasst das Verfahren den zusätzlichen Verfahrensschritt e):
e) Zugab von Essigsäure.

In einer Variante des Verfahrens umfasst das Verfahren den zusätzlichen Verfahrensschritt f):
f) Zugab von Wasser.

In einer Variante des Verfahrens umfasst das Verfahren den zusätzlichen Verfahrensschritt g):
g) Zugabe von p-Toluolsulfonsäure (PTSA).

In einer Variante des Verfahrens wird das Reaktionsgemisch im Verfahrensschritt d) auf eine Temperatur im Bereich von 80 °C bis 160 °C erhitzt.

In einer bevorzugten Variante des Verfahrens wird das Reaktionsgemisch im Verfahrensschritt d) auf eine Temperatur im Bereich von 100 °C bis 140 °C erhitzt.

In einer Variante des Verfahrens erfolgt das Zuführen von CO im Verfahrensschritt c) so, dass die Reaktion bei einem CO-Druck im Bereich von 20 bar bis 60 bar verläuft.

In einer bevorzugten Variante des Verfahrens erfolgt das Zuführen von CO im Verfahrensschritt c) so, dass die Reaktion bei einem CO-Druck im Bereich von 30 bar bis 50 bar verläuft.

In einer Variante des Verfahrens wird das Diisobuten zur Verbindung **P1** umgesetzt:

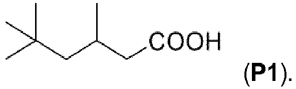

Im Folgenden wird die Erfindung anhand eines Ausführungsbeispiels näher erläutert.

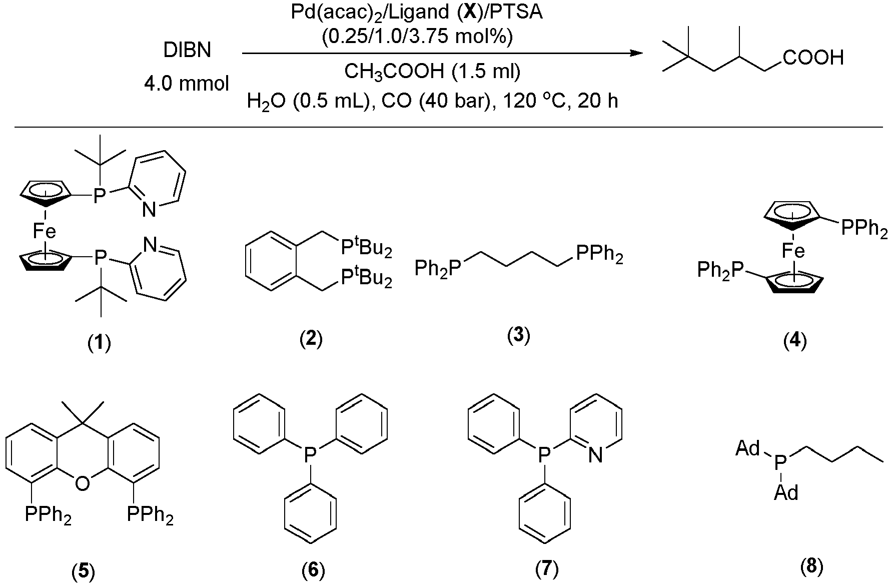

Ein 4-mL-Vial wurde mit [Pd(acac)₂] (3,05 mg, 0,25 mol-%), Ligand (X) (20,64 mg, 1,0 mol-%), p-Toluolsulfonsäure (28.5 mg, 3.75 mol-%) und einem im Ofen getrockneten Rührstab beschickt. Dann wird das Vial mit Septen (PTFE-beschichteter Styrol-Butadien-Kautschuk) und Phenolharzdeckel verschlossen. Das Vial wird dreimal evakuiert und mit Argon wieder befüllt. Mit einer Spritze wurden H₂O (0,5 ml), Essigsäure (1,5 ml) und Diisobuten (DIBN) (4,0 mmol) in das Vial gegeben. Das Vial wurde in eine Legierungsplatte gestellt, die in einen Autoklaven (300 ml) der Reihe 4560 von Parr Instruments unter Argonatmosphäre überführt wurde. Nach dreimaligem Spülen des Autoklaven mit CO wurde der CO-Druck auf 40 bar bei Raumtemperatur erhöht. Die Reaktion wurde 20 h bei 120 °C durchgeführt. Nach Beendigung der Reaktion wurde der Autoklav auf Raumtemperatur abgekühlt und vorsichtig entspannt. Dann wurde Isooctan (100 ul) als interner Standard zugegeben. Der Umsatz wurde durch GC-Analyse gemessen.

Der oben beschriebene Versuch wurde unter Variation des Liganden (**X**) mit **X** = **1** bis **8** durchgeführt.

Die Ergebnisse sind in der nachfolgenden Tabelle zusammengestellt.

| Ligand | Ausbeute (%) |
|---|---|
| (**1**)* | > 99 |
| (**2**) | 7 |
| (**3**) | 39 |
| (**4**) | 26 |
| (**5**) | 16 |
| (**6**) | 8 |
| (**7**) | 13 |
| (**8**) | 29 |

| | |
|---|---|
| * erfindungsgemäßes Verfahren | |

Wie die Versuchsergebnisse zeigen, wird die Aufgabe durch ein erfindungsgemäßes Verfahren gelöst.

## Patentansprüche

1. Verfahren umfassend die Verfahrensschritte:
a) Zugabe von Diisobuten;
b) Zugabe eines Komplexes, welcher eine Verbindung gemäß der allgemeinen Formel (I) umfasst, sowie Pd,
oder einer Verbindung gemäß der allgemeinen Formel (I) und eine Substanz, welche Pd umfasst 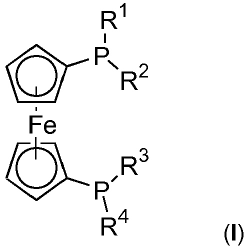 wobei
R¹, R², R³, R⁴ jeweils unabhängig voneinander ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl, -(C₃-C₂₀)-Heteroaryl;
mindestens einer der Reste R¹, R², R³, R⁴ für einen -(C₆-C₂₀)-Heteroarylrest mit mindestens sechs Ringatomen steht;
und
R¹, R², R³, R⁴, falls diese für -(C₁-C₁₂)-Alkyl, -(C₃-C₁₂)-Cycloalkyl, -(C3-C12)-Heterocycloalkyl, -(C₆-C₂₀)-Aryl, -(C₃-C₂₀)-Heteroaryl oder -(C₆-C₂₀)-Heteroaryl stehen,
jeweils unabhängig voneinander mit einem oder mehreren Substituenten ausgewählt aus
-(C₁-C₁₂)-Alkyl, -(C₃-C₁₂)-Cycloalkyl, -(C₃-C₁₂)-Heterocycloalkyl, -O-(C₁-C₁₂)-Alkyl,-O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H,-NH₂, Halogen substituiert sein können;
c) Zuführen von CO;
d) Erhitzen des Reaktionsgemisches, so dass das Diisobuten zu einer Carbonsäure umgesetzt wird,
wobei das Diisobuten direkt zur Carbonsäure umgesetzt wird.

2. Verfahren nach Anspruch 1,
wobei mindestens zwei der Reste R¹, R², R³, R⁴ für einen -(C₆-C₂₀)-Heteroarylrest mit mindestens sechs Ringatomen stehen.

3. Verfahren nach einem der Ansprüche 1 oder 2,
wobei die Reste R¹ und R³ jeweils für einen -(C₆-C₂₀)-Heteroarylrest mit mindestens sechs Ringatomen stehen.

4. Verfahren nach einem der Ansprüche 1 bis 3,
wobei die Reste R¹ und R³ jeweils für einen -(C₆-C₂₀)-Heteroarylrest mit mindestens sechs Ringatomen stehen;
R² für -(C₆-C₂₀)-Heteroaryl mit mindestens sechs Ringatomen steht oder ausgewählt ist aus -(C₁-C₁₂)-Alkyl, -(C₃-C₁₂)-Cycloalkyl, -(C₃-C₁₂)-Heterocycloalkyl, -(C₆-C₂₀)-Aryl;
und R⁴ ausgewählt ist aus -(C₁-C₁₂)-Alkyl, -(C₃-C₁₂)-Cycloalkyl, -(C₃-C₁₂)-Heterocycloalkyl, -(C₆-C₂₀)-Aryl.

5. Verfahren nach einem der Ansprüche 1 bis 4,
wobei die Reste R¹ und R³ jeweils für einen -(C₆-C₂₀)-Heteroarylrest mit mindestens sechs Ringatomen stehen;
und R² und R⁴ ausgewählt sind aus -(C₁-C₁₂)-Alkyl, -(C₃-C₁₂)-Cycloalkyl, -(C₃-C₁₂)-Heterocycloalkyl, -(C₆-C₂₀)-Aryl.

6. Verfahren nach einem der Ansprüche 1 bis 5,
wobei die Reste R¹ und R³ jeweils für einen -(C₆-C₂₀)-Heteroarylrest mit mindestens sechs Ringatomen stehen;
und R² und R⁴ für -(C₁-C₁₂)-Alkyl stehen.

7. Verfahren nach einem der Ansprüche 1 bis 6,
wobei R¹, R², R³, R⁴, falls diese für einen Heteroarylrest stehen, jeweils unabhängig voneinander ausgewählt sind aus Pyridyl, Pyridazinyl, Pyrimidyl, Pyrazinyl, Benzofuranyl, Indolyl, Isoindolyl, Benzimidazolyl, Chinolyl, Isochinolyl.

8. Verfahren nach einem der Ansprüche 1 bis 7, wobei die Verbindung (I) die Struktur (1) aufweist:

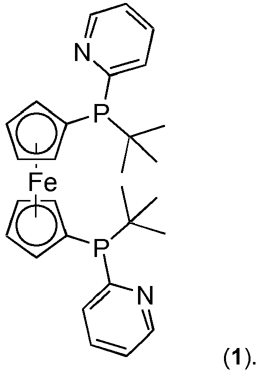

9. Verfahren nach einem der Ansprüche 1 bis 8,
wobei die Substanz im Verfahrensschritt b) ausgewählt ist aus:
PdCl₂, PdBr₂, Pd(acac)₂, Pd(dba)₂ (dba = Dibenzylidenaceton), PdCl₂(CH₃CN)₂.

10. Verfahren nach einem der Ansprüche 1 bis 9,
wobei das Verfahren den zusätzlichen Verfahrensschritt e) umfasst: e) Zugab von Essigsäure.

11. Verfahren nach einem der Ansprüche 1 bis 10,
wobei das Verfahren den zusätzlichen Verfahrensschritt f) umfasst: f) Zugab von Wasser.

12. Verfahren nach einem der Ansprüche 1 bis 11,
wobei das Verfahren den zusätzlichen Verfahrensschritt g) umfasst: g) Zugabe von p-Toluolsulfonsäure.

13. Verfahren nach einem der Verfahrensschritte 1 bis 12,
wobei das Reaktionsgemisch im Verfahrensschritt d) auf eine Temperatur im Bereich von 80 °C bis 160 °C erhitzt wird.

14. Verfahren nach einem der Verfahrensschritte 1 bis 13,
wobei das Zuführen von CO im Verfahrensschritt c) so erfolgt, dass die Reaktion bei einem CO-Druck im Bereich von 20 bar bis 60 bar verläuft.

15. Verfahren nach einem der Verfahrensschritte 1 bis 14, wobei das Diisobuten zur Verbindung **P1** umgesetzt wird:

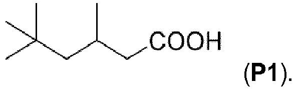

## Claims

1. Process comprising the process steps of:
a) addition of diisobutene;
b) addition of a complex, comprising a compound of general formula (**I**) and also Pd,
or a compound according to general formula (**I**) and a substance comprising Pd 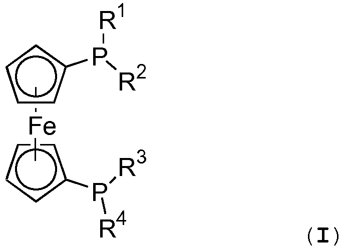 wherein
R¹, R², R³, R⁴ are each independently selected from -(C₁-C₁₂)-alkyl, -(C₃-C₁₂)-cycloalkyl, -(C₃-C₁₂)-heterocycloalkyl, -(C₆-C₂₀)-aryl, -(C₃-C₂₀)-heteroaryl;
at least one of the R¹, R², R³, R⁴ radicals is a-(C₆-C₂₀)-heteroaryl radical having at least six ring atoms;
and
R¹, R², R³, R⁴, if they are -(C₁-C₁₂)-alkyl, -(C₃-C₁₂)-cycloalkyl, -(C₃-C₁₂) -heterocycloalkyl, -(C₆-C₂₀)-aryl, -(C₃-C₂₀) -heteroaryl or -(C₆-C₂₀)-heteroaryl, may each independently be substituted by one or more substituents selected from
-(C₁-C₁₂) -alkyl, -(C₃-C₁₂)-cycloalkyl, -(C₃-C₁₂)-heterocycloalkyl, -O-(C₁-C₁₂) -alkyl, -O-(C₁-C₁₂)-alkyl- (C₆-C₂₀) -aryl, -O-(C₃-C₁₂) -cycloalkyl, -S-(C₁-C₁₂)-alkyl, -S-(C₃-C₁₂) -cycloalkyl, -COO-(C₁-C₁₂)-alkyl, -COO-(C₃-C₁₂) -cycloalkyl, -CONH-(C₁-C₁₂)-alkyl, -CONH-(C₃-C₁₂)-cycloalkyl, -CO-(C₁-C₁₂)-alkyl, -CO-(CᵣC₁₂)-cycloalkyl, -N-[(C₁-C₁₂) -alkyl]₂, -(C₆-C₂₀)-aryl, -(C₆-C₂₀) -aryl-(C₁-C₁₂) -alkyl, -(C₆-C₂₀)-aryl-O- (C₁-C₁₂) -alkyl, -(C₃-C₂₀) -heteroaryl, -(C₃-C₂₀)-heteroaryl- (C₁-C₁₂) -alkyl, -(C₃-C₂₀) -heteroaryl-O-(C₁-C₁₂)-alkyl, -COOH, -OH, -SO₃H,-NH₂, halogen;
c) feeding in CO;
d) heating the reaction mixture such that the diisobutene is converted to a carboxylic acid, wherein the diisobutene is directly converted to the carboxylic acid.

2. Process according to Claim 1,
wherein at least two of the R¹, R², R³, R⁴ radicals are a -(C₆-C₂₀)-heteroaryl radical having at least six ring atoms.

3. Process according to either of Claims 1 and 2, wherein the R¹ and R³ radicals are each a -(C₆-C₂₀)-heteroaryl radical having at least six ring atoms.

4. Process according to any of Claims 1 to 3,
wherein the R¹ and R³ radicals are each a -(C₆-C₂₀)-heteroaryl radical having at least six ring atoms; R² is -(C₆-C₂₀)-heteroaryl having at least six ring atoms or is selected from -(C₁-C₁₂)-alkyl, -(C₃-C₁₂)-cycloalkyl, -(C₃-C₁₂) -heterocycloalkyl, -(C₆-C₂₀)-aryl;
and R⁴ is selected from -(C₁-C₁₂)-alkyl, -(C₃-C₁₂)-cycloalkyl, -(C₃-C₁₂) -heterocycloalkyl, -(C₆-C₂₀)-aryl.

5. Process according to any of Claims 1 to 4,
wherein the R¹ and R³ radicals are each a -(C₆-C₂₀)-heteroaryl radical having at least six ring atoms; and R² and R⁴ are selected from -(C₁-C₁₂) -alkyl,-(C₃-C₁₂) -cycloalkyl, -(C₃-C₁₂) -heterocycloalkyl,-(C₆-C₂₀) -aryl.

6. Process according to any of Claims 1 to 5,
wherein the R¹ and R³ radicals are each a -(C₆-C₂₀)-heteroaryl radical having at least six ring atoms; and R² and R⁴ are -(C₁-C₁₂) -alkyl.

7. Process according to any of Claims 1 to 6,
wherein R¹, R², R³, R⁴, if they are a heteroaryl radical, are each independently selected from pyridyl, pyridazinyl, pyrimidyl, pyrazinyl, benzofuranyl, indolyl, isoindolyl, benzimidazolyl, quinolyl, isoquinolyl.

8. Process according to any of Claims 1 to 7, wherein the compound (I) has the structure (1):

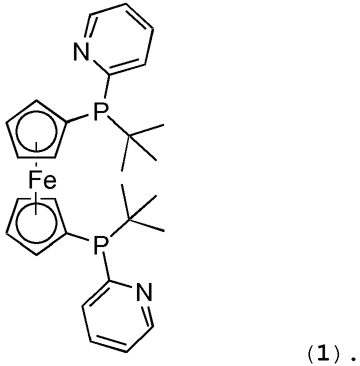

9. Process according to any of Claims 1 to 8,
wherein the substance in process step b) is selected from:
PdCl₂, PdBr₂, Pd(acac)₂, Pd(dba)₂ (dba = dibenzylideneacetone), PdCl₂ (CH₃CN)₂.

10. Process according to any of Claims 1 to 9,
wherein the process comprises the additional process step e) :
e) addition of acetic acid.

11. Process according to any of Claims 1 to 10,
wherein the process comprises the additional process step f):
f) addition of water.

12. Process according to any of Claims 1 to 11,
wherein the process comprises the additional process step g) :
g) addition of p-toluenesulfonic acid.

13. Process according to any of process steps 1 to 12, wherein the reaction mixture is heated to a temperature in the range from 80°C to 160°C in process step d).

14. Process according to any of process steps 1 to 13, wherein the CO is fed in in process step c) such that the reaction proceeds under a CO pressure in the range from 20 bar to 60 bar.

15. Process according to any of process steps 1 to 14, wherein the diisobutene is converted to the compound **P1:**

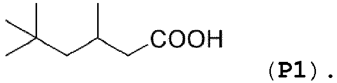

## Revendications

1. Procédé comprenant les étapes de procédé :
a) ajout de diisobutène ;
b) ajout d'un complexe, qui comprend un composé selon la formule générale (I), ainsi que du Pd, ou d'un composé selon la formule générale (I) et d'une substance qui comprend du Pd 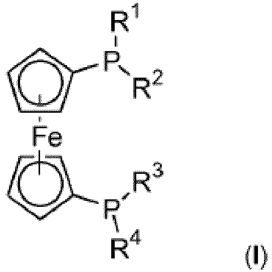
R¹, R², R³, R⁴ à chaque fois indépendamment les uns des autres, étant choisis parmi -C₁-₁₂-alkyle, -C₃₋₁₂-cycloalkyle, -C₃₋₁₂-hétérocycloalkyle, -C₆₋₂₀-aryle, -C₃₋₂₀-hétéroaryle ;
au moins un des radicaux R¹, R², R³, R⁴ représentant un radical -C₆₋₂₀-hétéroaryle comportant au moins six atomes de cycle ;
et
R¹, R², R³, R⁴, dans le cas où ils représentent-C₁₋₁₂-alkyle, -C₃₋₁₂-cycloalkyle, -C₃₋₁₂-hétérocycloalkyle, -C₆₋₂₀-aryle, -C₃₋₂₀-hétéroaryle ou -C₆₋₂₀-hétéroaryle,
pouvant être substitués, à chaque fois indépendamment les uns des autres, par un ou plusieurs substituants choisis parmi
-C₁₋₁₂-alkyle, -C₃₋₁₂-cycloalkyle, -C₃₋₁₂-hétérocycloalkyle, -O-C₁₋₁₂-alkyle, -O-C₁₋₁₂-alkyl-C₆₋₂₀-aryle, -O-C₃₋₁₂-cycloalkyle, -S-C₁₋₁₂-alkyle, -S-C₃₋₁₂-cycloalkyle, -COO-C₁₋₁₂-alkyle,-COO-C₃₋₁₂-cycloalkyle, -CONH-C₁₋₁₂-alkyle, -CONH-C₃₋₁₂-cycloalkyle, -CO-C₁₋₁₂-alkyle, -CO-C₃₋₁₂-cycloalkyle, -N-[C₁₋₁₂-alkyl]₂, -C₆₋₂₀-aryle, -C₆₋₂₀-aryl-C₁₋₁₂-alkyle, -C₆₋₂₀-aryl-O-C₁₋₁₂-alkyle,-C₃₋₂₀-hétéroaryle, -C₃₋₂₀-hétéroaryl-C₁₋₁₂-alkyle,-C₃₋₂₀-hétéroaryl-O-C₁₋₁₂-alkyle, -COOH, -OH, -SO₃H, -NH₂, halogène ;
c) introduction de CO ;
d) chauffage du mélange réactionnel, de sorte que le diisobutène est transformé en un acide carboxylique,
le diisobutène étant directement transformé en l'acide carboxylique.

2. Procédé selon la revendication 1,
au moins deux des radicaux R¹, R², R³, R⁴ représentant un radical -C₆₋₂₀-hétéroaryle comportant au moins six atomes de cycle.

3. Procédé selon l'une quelconque des revendications 1 et 2,
les radicaux R¹ et R³ représentant à chaque fois un radical -C₆₋₂₀-hétéroaryle comportant au moins six atomes de cycle.

4. Procédé selon l'une quelconque des revendications 1 à 3,
les radicaux R¹ et R³ représentant à chaque fois un radical -C₆₋₂₀-hétéroaryle comportant au moins six atomes de cycle ;
R² représentant -C₆₋₂₀-hétéroaryle comportant au moins six atomes de cycle ou étant choisi parmi-C₁₋₁₂-alkyle, -C₃₋₁₂-cycloalkyle, -C₃₋₁₂-hétérocycloalkyle, -C₆₋₂₀-aryle ;
et R⁴ étant choisi parmi -C₁₋₁₂-alkyle, -C₃₋₁₂-cycloalkyle, -C₃₋₁₂-hétérocycloalkyle, -C₆₋₂₀-aryle.

5. Procédé selon l'une quelconque des revendications 1 à 4,
les radicaux R¹ et R³ représentant à chaque fois un radical -C₆₋₂₀-hétéroaryle comportant au moins six atomes de cycle ;
et R² et R⁴ étant choisis parmi -C₁₋₁₂-alkyle, -C₃₋₁₂-cycloalkyle, -C₃₋₁₂-hétérocycloalkyle, -C₆₋₂₀-aryle.

6. Procédé selon l'une quelconque des revendications 1 à 5,
les radicaux R¹ et R³ représentant à chaque fois un radical -C₆₋₂₀-hétéroaryle comportant au moins six atomes de cycle ;
et R² et R⁴ représentant -C₁₋₁₂-alkyle.

7. Procédé selon l'une quelconque des revendications 1 à 6,
R¹, R², R³, R⁴, dans le cas où ceux-ci représentent un radical hétéroaryle, étant choisis à chaque fois indépendamment les uns des autres parmi pyridinyle, pyridazinyle, pyrimidinyle, pyrazinyle, benzofurannyle, indolyle, isoindolyle, benzimidazolyle, quinoléinyle, isoquinoléinyle.

8. Procédé selon l'une quelconque des revendications 1 à 7,
le composé (I) présentant la structure (1) :

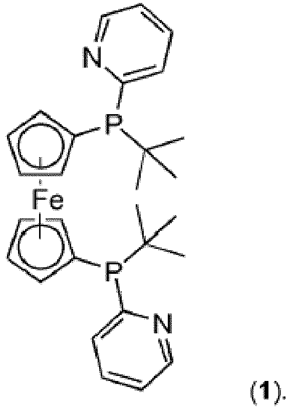

9. Procédé selon l'une quelconque des revendications 1 à 8, la substance dans l'étape de procédé b) étant choisie parmi :
PdCl₂, PdBr₂, Pd(acac)₂, Pd(dba)₂ (dba = dibenzylidèneacétone), PdCl₂(CH₃CN)₂.

10. Procédé selon l'une quelconque des revendications 1 à 9,
le procédé comprenant l'étape de procédé supplémentaire e) :
e) ajout d'acide acétique.

11. Procédé selon l'une quelconque des revendications 1 à 10,
le procédé comprenant l'étape de procédé supplémentaire f) :
f) ajout d'eau.

12. Procédé selon l'une quelconque des revendications 1 à 11,
le procédé comprenant l'étape de procédé supplémentaire g) :
g) ajout d'acide p-toluènesulfonique.

13. Procédé selon l'une quelconque des étapes de procédé 1 à 12, le mélange réactionnel dans l'étape de procédé d) étant chauffé à une température dans la plage de 80 °C à 160 °C.

14. Procédé selon l'une quelconque des étapes de procédé 1 à 13, l'introduction de CO dans l'étape de procédé c) étant réalisée de telle façon, que la réaction a lieu à une pression de CO dans la plage de 20 bars à 60 bars.

15. Procédé selon l'une quelconque des étapes de procédé 1 à 14,
le diisobutène étant transformé en composé P1 :
